# EUROPÄISCHE PATENTSCHRIFT

(11) **EP 1 263 706 B1**
(45) Veröffentlichungstag und Bekanntmachung des Hinweises auf die Patenterteilung: **09.11.2005**
(21) Anmeldenummer: 01919320.0
(22) Anmeldetag: 21.02.2001
(51) Int. Cl.: C07C 51/235, C07C 53/124, C07C 53/126, C07C 53/122

(54) **VERFAHREN ZUR HERSTELLUNG GERADKETTIGER ALIPHATISCHER CARBONSÄUREN AUS ALDEHYDEN**
METHOD FOR THE SYNTHESIS OF STRAIGHT-CHAIN ALIPHATIC CARBOXYLIC ACIDS FROM ALDEHYDES
PROCEDE DE FABRICATION D'ACIDES CARBOXYLIQUES ALIPHATIQUES LINEAIRES A PARTIR D'ALDEHYDES

(30) Priorität: 04.03.2000 DE 10010770
(43) Veröffentlichungstag der Anmeldung: 11.12.2002
(73) Patentinhaber: Celanese Chemicals Europe GmbH, 60439 Frankfurt am Main (DE)
(72) Erfinder: SPRINGER, Helmut, 46539 Dinslaken (DE)
(86) Internationale Anmeldenummer: PCT/EP2001/001940
(87) Internationale Veröffentlichungsnummer: WO 2001/066503

(56) Entgegenhaltungen:
- DE-B- 1 083 800
- DE-B- 1 154 454

## Beschreibung

Die vorliegende Erfindung betrifft ein neues, nichtkatalytisches Verfahren zur Herstellung geradkettiger aliphatischer Carbonsäuren aus Aldehyden durch Oxidation mit Sauerstoff oder Sauerstoff enthaltenden Gasgemischen.

Aldehyde werden in großem Umfang als Ausgangsstoffe zur Gewinnung von Carbonsäuren verwendet. Die Vorzugsstellung für dieses Einsatzgebiet verdanken Aldehyde ihrer hohen Verfügbarkeit nach einer Reihe, auch industriell genutzter Prozesse. Überdies läßt sich die Carbonylgruppe der Aldehyde leicht in die für Carbonsäuren charakteristische Carboxylgruppe umwandeln. Im Rahmen technisch ausgeübter Verfahren erfolgt die Umsetzung von Aldehyden zu Carbonsäuren vorwiegend in Anwesenheit von Katalysatoren. Es sind jedoch auch Prozesse bekannt, bei denen auf die Verwendung von Katalysatoren verzichtet wird. Zur Vermeidung von Nebenreaktionen arbeitet man sowohl bei den katalytischen als auch bei den nichtkatalytischen Prozessen bei möglichst niedrigen Temperaturen, im allgemeinen wird eine Reaktionstemperatur von 100°C nicht überschritten. Als Katalysatoren kommen vorwiegend Salze von Übergangsmetallen in Betracht, insbesondere Salze des Kobalts und des Mangans sowie des Chroms, Eisens, Kupfers, Nickels, Silbers und Vanadiums. Häufig ist die Carbonsäurebildung aus Aldehyden, selbst bei Einhaltung optimaler Temperaturbedingungen, mit Neben- und Abbaureaktionen verbunden. Das gilt gleichermaßen für Reaktionen in Gegenwart wie in Abwesenheit von Katalysatoren. In solchen Fällen kann die Selektivität der Umsetzung durch Zusatz von Alkalimetallsalzen schwacher Säuren zu den Reaktanten erheblich verbessert werden. Nachteilig bei dieser Verfahrensvariante ist jedoch, dass die Salze inhibierend wirken, so dass für eine vollständige Umsetzung der Ausgangsstoffe lange Reaktionszeiten erforderlich sind.

Nach dem in der DE-OS 30 29 700 beschriebenen Verfahren werden zur Herstellung von aliphatischen Monocarbonsäuren mit 6 bis 9 Kohlenstoffatomen die entsprechenden Aldehyde mit Sauerstoff in reiner Form oder mit Luft oxidiert. Als Katalysator wirkt eine Kombination von Mangan- und Kupferverbindungen, die in der Säure löslich sind, wobei das Molverhältnis von Mangan zu Kupfer im Bereich von 5:1 bis 0,5:1 liegt. Die Umsetzung der Ausgangsstoffe erfolgt in flüssiger Phase bei Temperaturen von etwa 50 bis 80°C und Drücken im Bereich von etwa 1,4 bis 10,3 bar. Als Hauptschwierigkeit dieses Verfahrens wird in der Prozessbeschreibung die Anwesenheit von Kupfer- und auch Manganverbindungen im Reaktionsprodukt, d.h. in der Carbonsäure, geschildert. Zur Entfernung der Metalle sind aufwendige Reinigungsmaßnahmen erforderlich, beispielsweise ihre Ausfällung mit wäßriger Oxalsäure.

Das in der US-Patentschrift 4 487 720 offenbarte Verfahren zur Herstellung von C₅- bis C₉-Monocarbonsäuren durch Oxidation von Aldehyden der gleichen Kohlenstoffatom-Zahl mit reinem Sauerstoff oder mit Luft arbeitet ebenfalls mit Kupfer- und Manganverbindungen als Katalysatoren. Als Nachteil dieser Arbeitsweise wird die Bildung von Kupferfilmen beschrieben, die bei der destillativen Reinigung der Säure auftreten und mechanische Schäden in der Destillationsapparatur zur Folge haben. Um dieses Problem zu vermeiden wird empfohlen, die Destillation in Gegenwart von Sauerstoff durchzuführen.

Ein weiteres katalytisches Verfahren zur Reaktion von Aldehyden mit Sauerstoff unter Bildung von Carbonsäuren ist Gegenstand der offengelegten internationalen Anmeldung WO97/14668. Als Katalysatoren finden substituierte oder unsubstituierte Alkylamine, Alkylamin-N-oxide, aromatische Amine, aromatische N-oxide, heterocyclische Amine, heterocyclische Amin-N-oxide und deren Mischungen Verwendung. Ausdrücklich wird darauf hingewiesen, dass die katalytisch wirkenden Stickstoffverbindungen einen im Vergleich zum Reaktionsprodukt höheren Siedepunkt besitzen müssen, um eine Verunreinigung der Säure durch den Katalysator zu unterbinden.

Nach der Lehre der offengelegten japanischen Patentanmeldung 53-105413 oxidiert man α-verzweigte aliphatische Aldehyde mit Sauerstoff in Gegenwart von Lithium- oder Erdalkalimetallverbindungen, die in Mengen von 0,01 bis 10 Gew.-% (bezogen auf das gesamte Reaktionssystem) eingesetzt werden, um α-verzweigte aliphatische Carbonsäuren herzustellen.

Kennzeichnend für die in der französischen Patentanmeldung 2 769 624 beschriebene Arbeitsweise ist die Einhaltung niedriger Reaktionstemperaturen, nämlich Temperaturen zwischen 0 und 25°C. Das Verfahren erfordert ebenfalls die Gegenwart von Alkalimetall- bzw. Erdalkalimetallverbindungen als Hilfsstoffe. Es bleibt offen, welche speziellen Wirkungen diese Verbindungen entfalten, d.h. ob sie, wie bekannt, lediglich die Selektivität der Umsetzung verbessern oder aber bei den gewählten, niedrigen Temperaturen, möglicherweise auch die Reaktionsgeschwindigkeit erhöhen.

Gemische isomerer verzweigter aliphatischer oder cycloaliphatischer Carbonsäuren erhält man entsprechend der Arbeitsweise der deutschen Patentschrift 1 154 454 aus Aldehyden, die in dünner, flüssiger Schicht bei einer Temperatur von 65 bis 110°C ohne Verwendung von Katalysatoren mit einem sauerstoffhaltigen Gas oxidiert werden. Die Umsetzung wird in leeren oder mit inerten Stoffen großer Oberfläche gefüllten Rohren durchgeführt. Der Säureanteil im Reaktionsprodukt beträgt zwischen 59 und 80%.

Übereinstimmend setzt man nach den bekannten Oxidationsverfahren zur Herstellung von Carbonsäuren als Ausgangsmaterial strukturell einheitliche Aldehyde ein, d.h entweder geradkettige oder verzweigte Verbindungen derselben Molekülgröße (Kohlenstoffatom-Zahl). Ein wichtiger Grund für die Verwendung weitgehend reiner Carbonylverbindungen zur Weiterverarbeitung ist das Bemühen, zusätzliche Verfahrensschritte in Form von Trennoperationen zu vermeiden. Überdies ist man bestrebt, störend wirkende Bestandteile, die den Oxidationsprozess belasten und/oder die Qualität des Reaktionsproduktes beeinträchtigen, auszuschließen.

Dementsprechend sind eine Vielzahl von Verfahren bekannt, um Gemische isomerer Aldehyde, die auch noch weitere Bestandteile, z.B. Katalysatoren, Aldehydfolgeprodukte wie Alkohole, Säuren und Kondensationsprodukte enthalten können, in die reinen Komponenten aufzutrennen. Eine häufig angewandte, wegen der thermischen Empfindlichkeit der Aldehyde jedoch nicht unproblematische Arbeitsweise ist die Destillation. Um die Belastung der Aldehyde so gering wie möglich zu halten, schaltet man daher zweckmäßig mehrere Destillationskolonnen hintereinander und führt die Destillation bei möglichst kurzer Verweilzeit durch. In manchen Fällen gelingen Trennung und Reindarstellung der Aldehyde auch durch Azeotropdestillation mit Wasser oder anderen Schleppmitteln.

Insbesondere in der Laborpraxis erfolgt die Aldehydreinigung oft über Additions- und Kondensationsprodukte, die nach dem Trennschritt unter Rückbildung der Aldehyde gespalten werden. Beispiele für Additions- und Kondensationsverbindungen der Aldehyde sind Hydrazone, Bisulfit-Addukte (vgl. DE 24 59 152) und Acetale.

Die Reindarstellung von höheren n-Alkanalen aus n/iso-Aldehyd-Gemischen, die durch Hydroformylierung gewonnen wurden, kann durch Behandlung des Aldehyd-Gemischs mit nicht-oxidierenden Säuren erfolgen. Die dabei anfallenden 1,3,5-Trioxane der geradkettigen Aldehyde werden durch fraktionierte Kristallisation abgetrennt (vgl. DE 22 18 305).

Die bekannten Verfahren zur Herstellung von Carbonsäuren aus Aldehyden erfüllen nicht allein wegen der Empfehlung, vorzugsweise Alkanale gleicher Molekülgröße und -struktur einzusetzen, nicht die technischen und wirtschaftlichen Voraussetzungen, die an moderne, industriell genutzte Prozesse gestellt werden. Darüber hinaus ist die Anwendung von Katalysatoren auch mit aufwendigen Reinigungsschritten verbunden, denen das Reaktionsprodukt unterworfen werden muss, um Carbonsäuren zu erhalten, die problemlos weiterverarbeitet werden können. Nichtkatalytische Prozesse befriedigen häufig nicht hinsichtlich Umsatz und Selektivität zum gewünschten Produkt.

Es bestand daher die Aufgabe eine Arbeitsweise zu entwickeln, die die aufgezeigten Nachteile vermeidet und die Gewinnung von Carbonsäuren aus Aldehyden mit vertretbarem technischen Aufwand in hoher Ausbeute ermöglicht.

Diese Aufgabe wird gelöst durch ein Verfahren zur Herstellung geradkettiger aliphatischer Carbonsäuren mit 4 bis 11 Kohlenstoffatomen durch Oxidation der entsprechenden geradkettigten Aldehyde mit Sauerstoff oder Sauerstoff enthaltenden Gasgemischen bei 20 bis 100°C. Es ist dadurch gekennzeichnet, dass die Oxidation der geradkettigen Aldehyde in Gegenwart von 1 bis 30 mol-% verzweigten Aldehyden je mol geradkettigen Aldehyden erfolgt.

Überraschenderweise gelingt es durch Zugabe verzweigter Aldehyde in das Reaktionsgemisch die Oxidation von n-Aldehyden zu den entsprechenden Carbonsäuren mit reinem Sauerstoff oder Sauerstoff als Bestandteil von Gasgemischen, mit hohem Umsatz, sehr selektiv und ohne Einsatz von Katalysatoren durchzuführen.

Die dem Oxidationsgemisch erfindungsgemäß zugesetzten verzweigten Aldehyde sind vorzugsweise gesättigt und enthalten 3 bis 15, insbesondere 4 bis 11 Kohlenstoffatome im Molekül. Die Verwendung ungesättigter verzweigter Aldehyde ist zwar nicht ausgeschlossen, doch wird sie sich auf Ausnahmefälle beschränken. Hierbei ist zu berücksichtigen, dass ungesättigte Verbindungen eher als gesättigte Verbindungen im oxidierenden Milieu zur Bildung Sauerstoff enthaltender Abbauprodukte neigen. Derartige Nebenreaktionen sind unerwünscht, weil sie zur Verunreinigung der n-Carbonsäuren führen können.

Unter dem Begriff verzweigte Aldehyde werden entsprechend den gültigen Nomenklaturregeln Alkanale verstanden, die neben einer aus aneinandergereihten Kohlenstoffatomen bestehenden Hauptkette noch ein oder mehrere Kohlenstoffatome enthaltende Seitenketten aufweisen. Die Lage dieser Seitenketten im Molekül, ihre Anzahl und ihre Länge ist beliebig. Jedoch setzt man wegen ihrer leichten Zugänglichkeit bevorzugt Aldehyde ein, deren Seitenkette sich am α- oder β-Kohlenstoffatom (d.h. in 2- oder 3-Stellung), bezogen auf die Carbonylgruppe, befindet. Üblicherweise enthält der verzweigte Aldehyd lediglich eine Seitenkette. Sind im Molekül mehrere Seitenketten vorhanden, so können sie gleich oder unterschiedlich viele Kohlenstoffatome enthalten. Bevorzugt als Seitenketten sind der Methyl- und der Ethylrest. Beispiele für verzweigte Aldehyde sind iso-Butyraldehyd, 2-Methylbutanal, 3-Methylbutanal (iso-Valeraldehyd), 2-Methylpentanal, 2-Methylhexanal, 2-Ethylhexanal, 2-Methyloctanal, 3,5,5-Trimethylhexanal.

Je mol unverzweigten Aldehyds setzt man dem Reaktionsgemisch 1 bis 30 mol-%, vorzugsweise 2 bis 15 mol-% und insbesondere 5 bis 10 mol-% verzweigten Aldehyd zu. Besonders bewährt haben sich α- und β-verzweigte Aldehyde. Verglichen mit diesen sind Aldehyde, deren Seitenkette einen größeren Abstand zur Carbonylgruppe besitzen, weniger wirksam.

Die Molekülgröße von geradkettigem und verzweigtem Aldehyd braucht nicht übereinzustimmen. Es ist auch nicht erforderlich, verzweigte Aldehyde einheitlicher Struktur und/oder Molekülgröße einzusetzen. Vielmehr können auch Gemische aus zwei oder mehr unterschiedlichen verzweigten Aldehyden verwendet werden. Man ist daher in der Lage, nicht nur solche verzweigte Aldehyde zur Durchführung der Oxidationsreaktion auszuwählen, die sich, in Form der aus ihnen gebildeten verzweigten Säuren, leicht vom Zielprodukt abtrennen lassen, sondern auch wirtschaftlichen Erfordernissen, z.B. Verfügbarkeit und Preiswürdigkeit, Rechnung tragen.

Die Wirkungsweise der verzweigten Aldehyde im Oxidationsprozess wurde im einzelnen nicht untersucht. Zur Erklärung des Reaktionsmechanismus kann man annehmen, dass maßgebend für den Oxidationsvorgang die Konzentration von primär entstehenden Kohlenstoffradikalen im Reaktionsgemisch ist. Offensichtlich entstehen aus verzweigten Aldehyden leichter derartige Radikale als aus geradkettigen Aldehyden mit der Folge, dass in Gegenwart von verzweigten Aldehyden im Reaktionsgemisch freie Radikale in höherer Konzentration zur Verfügung stehen als bei Vorliegen von ausschließlich unverzweigten Aldehyden.

Normalerweise setzt man dem Reaktionsgemisch verzweigten Aldehyd in einer solchen Menge zu, dass sich das gewünschte Verhältnis geradkettiger/verzweigter Aldehyd einstellt. Umgekehrt kann man bei Vorliegen eines Überschusses an verzweigtem Aldehyd im Reaktionsgemisch entweder eine entsprechende Menge n-Aldehyd hinzufügen oder aber einen Teil des verzweigten Aldehyds abtrennen. Selbstverständlich sind auch Sonderfälle denkbar, in denen bereits aus dem Herstellungsprozess das für die Oxidationsstufe benötigte Aldehydgemisch vorliegt.

Das Verfahren der Erfindung wird im Temperaturbereich von 20 bis 100°C durchgeführt. Vorzugsweise arbeitet man zwischen 20 und 80°C, insbesondere zwischen 40 und 80°C. Die Temperaturführung, konstante oder variable Temperatur, kann den individuellen Erfordernissen des Ausgangsmaterials und den Reaktionsgegebenheiten angepasst werden.

Die Umsetzung der Reaktionspartner wird bevorzugt bei Atmosphärendruck durchgeführt. Die Anwendung erhöhten Drucks ist jedoch nicht ausgeschlossen. Üblicherweise arbeitet man in einem Bereich von Atmosphärendruck bis 1,0 MPa, vorzugsweise bei Atmosphärendruck bis 0,8 MPa.

Die zur Umwandlung von Aldehyden in Carbonsäuren nach dem erfindungsgemäßen Verfahren erforderliche Reaktionszeit hängt unter anderem ab von der Reaktionstemperatur, der Art der Einsatzstoffe und dem Mengenverhältnis der Reaktanten zueinander. Normalerweise beträgt sie 30 min bis 20 h, insbesondere 3 bis 8 h.

Im Mittelpunkt des neuen Prozesses steht die Oxidation von unverzweigten C₄- bis C₁₁-Aldehyden. Die Herkunft der Aldehyde ist nicht auf bestimmte Herstellungsverfahren beschränkt. Aufgrund ihrer leichten Zugänglichkeit werden durch Oxosynthese, d.h. durch Reaktion von C₃- bis C₁₀-Olefinen mit Kohlenmonoxid und Wasserstoff gewonnene Aldehyde bevorzugt. In diesem Zusammenhang ist es nicht entscheidend, welche spezielle Ausführungsform der Oxosynthese zur Gewinnung der Aldehyde diente, d.h. ob die Reaktion z.B. durch Kobalt oder durch Rhodium katalysiert wurde, ob man die Metalle allein oder zusammen mit Komplexbildnern einsetzte und der Katalysator im Reaktionsgemisch homogen gelöst war oder eine eigene, heterogene Phase bildete.

Als Oxidationsmittel verwendet man nach dem Verfahren der Erfindung molekularen Sauerstoff oder Gasgemische, die molekularen Sauerstoff enthalten. Weitere Bestandteile derartiger Gasgemische sind inerte Gase, z.B. Stickstoff, Edelgase und Kohlendioxid. Der Anteil der inerten Bestandteile des Sauerstoff enthaltenden Gasgemisches beträgt bis zu 90 Vol-%, insbesondere 30 bis 80 Vol-%. Die bevorzugten Oxidationsmittel sind Sauerstoff oder Luft.

Die Aldehyde können als solche oder gelöst in einem, unter den Reaktionsbedingungen inerten, Lösungsmittel eingesetzt werden. Beispiele für geeignete Lösungsmittel sind Ketone wie Aceton, Ester, z.B. Ethylacetat, Kohlenwasserstoffe, z.B. Toluol und Nitrokohlenwasserstoffe wie Nitrobenzol. Die Konzentration des Aldehyds wird durch seine Löslichkeit in dem Lösungsmittel begrenzt.

Das erfindungsgemäße Verfahren kann absatzweise oder kontinuierlich durchgeführt werden. Eine Rückführung nicht umgesetzter Reaktionsteilnehmer ist in beiden Fällen möglich.

Nach einer bewährten Ausführungsform des erfindungsgemässen Verfahrens legt man n-Aldehyd und verzweigten Aldehyd in einem geeigneten Reaktor, z.B. einem mit einem Anströmboden versehenen Rohrreaktor, der gegebenenfalls noch Füllkörper enthält, vor und leitet den Sauerstoff oder das Sauerstoff enthaltende Gasgemisch von unten durch den Aldehyd.

Entsprechend einer weiteren Ausführungsform verwendet man als Reaktor einen Rieselturm, der Füllkörper enthält: Über die Füllung läßt man den Aldehyd herabrieseln und leitet in den Turm gleichzeitig im Gleich- oder Gegenstrom, Sauerstoff oder ein Sauerstoff enthaltendes Gasgemisch ein. Unabhängig von der jeweiligen apparativen Ausgestaltung des erfindungsgemäßen Verfahrens muß das gewünschte Reaktionsprodukt, d.h. die geradkettige aliphatische Carbonsäure, von den Begleitstoffen, insbesondere von der aus dem verzweigten Aldehyd gebildeten verzweigten Carbonsäure, abgetrennt werden. Die Isolierung der geradkettigen Säure aus dem Reaktionsgemisch erfolgt nach konventionellen Verfahren. Geeignet sind die Destillation bei normalem oder vermindertem Druck und die Trägerdampfdestillation, beispielsweise in der gebräuchlichen Ausführungsform der Wasserdampfdestillation. Auch durch fraktionierte Kristallisation ist eine Trennung des Hauptprodukts von den zwangsweise anfallenden Begleitstoffen möglich. Welche Trennoperation im Einzelfall anzuwenden ist, richtet sich nach den individuellen Möglichkeiten und Erfordernissen. Es versteht sich von selbst, dass auch die im Verlauf des Oxidationsprozesses aus dem verzweigten Aldehyd gebildete verzweigte Carbonsäure unmittelbar oder nach chemischer Umwandlung als Wertprodukt weiter verwendet werden kann.

In den folgenden Beispielen wird die Herstellung von n-Pentansäure, n-Heptansäure und n-Nonansäure nach dem erfindungsgemäßen Verfahren beschrieben. Die Umsetzung der entsprechenden Ausgangsaldehyde läuft in Gegenwart von verzweigten Aldehyden als Reaktionshilfsmitteln ab. Den Beispielen werden die Ergebnisse von Vergleichsversuchen gegenübergestellt, in denen der n-Aldehyd allein, also in Abwesenheit von verzweigtem Aldehyd, oxidiert wurde. Die jeweiligen Versuchsergebnisse werden durch Angabe folgender Kenngrößen wiedergegeben:
- Auswaage an Oxidationsprodukt (Rohsäure); sie steht in einer Wechselbeziehung zum Umsatz;
- GC-Analyse der Rohsäure; die Vorlauf- und die Nachlaufkomponente sind nicht aufgegliedert, sondern unter den Bezeichnungen Leichtsieder und Hochsieder zusammengefasst;
- Aldehyd-Umsatz; unabhängig vom Zusatz eines verzweigten Aldehyds zum Reaktionsgemisch bezieht sich der Aldehyd-Umsatz ausschließlich auf die geradkettige Verbindung;
- Selektivität; sie ergibt sich aus dem n-Carbonsäure-Anteil im Reaktionsprodukt, bezogen auf umgesetzten n-Aldehyd.

Selbstverständlich ist das neue Verfahren nicht auf die nachstehend beschriebenen Ausführungsformen beschränkt.

### Beispiele

### Herstellung von n-Pentansäure

### Vergleichsbeispiel 1

Die Flüssigphasenoxidation von n-Pentanal zu n-Pentansäure wurde ohne Katalysatorzusatz in einem Blasensäulen-Reaktor aus Glas mit einem Innendurchmesser von 38 mm und 150 cm Länge durchgeführt. Abhängig vom Reaktionsverhalten wurde der Reaktor mantelseitig durch einen mit einem Wärmeaustauscher verbundenen Wasserkreislauf gekühlt oder beheizt und die Innentemperatur auf diese Weise konstant gehalten. Die Sauerstoffzufuhr erfolgte von unten durch eine mit der Blasensäule verbundene Glasfilterplatte mit einer maximalen Porenweite von 16-40µm.

In die Oxidationen wurden jeweils 800,0 g n-Pentanal folgender, gaschromatographisch bestimmter, Zusammensetzung eingesetzt:
0,17 % Leichtsieder
99,33 % n-Pentanal
0,42 % n-Pentansäure
0,08 % Hochsieder

Nach 6 Stunden Oxidation bei konstant 50°C wurden folgende Ergebnisse ermittelt:

| | |
|---|---|
| Auswaage Oxidationsprodukt (g) | 932,9 |
| GC-Analyse (%): | |
| Leichtsieder | 0,44 |
| n-Pentanal | 3,56 |
| n-Pentansäure | 95,24 |
| Hochsieder | 0,76 |
| Umsatz n-Pentanal (% d.Th.) | 96,3 |
| Selektivität zu n-Pentansäure (% d.Th.) | 99,0 |

### Beispiel 1

Dieser Versuch wurde unter den Bedingungen des Vergleichsbeispiels 1 durchgeführt mit der Abweichung, dass der Einsatzaldehyd neben 93,96 Gew.-% n-Pentanal 5,36 Gew.-% 2-Methylbutanal (entsprechend einem Molverhältnis von 100 zu 5,70) enthielt.

In die Oxidation wurden 800,0 g dieser Mischung eingesetzt.

Nach 6 Stunden Oxidation bei konstant 50°C wurden folgende Ergebnisse ermittelt:

| | |
|---|---|
| Auswaage Oxidationsprodukt (g) | 935,2 |
| GC-Analyse (%): | |
| Leichtsieder | 0,77 |
| 2-Methylbutanal | 0,06 |
| n-Pentanal | 2,75 |
| 2-Methylbuttersäure | 4,95 |
| n-Pentansäure | 91,02 |
| Hochsieder | 0,45 |
| | |
| Umsatz n-Pentanal (% d.Th.) | 97,00 |
| Selektivität zu n-Pentansäure (% d.Th.) | 99,40 |

### Beispiel 2

Dieser Versuch wurde unter den Bedingungen des Vergleichsbeispiels 1 durchgeführt mit der Abweichung, dass der Einsatzaldehyd neben 88,88 Gew.-% n-Pentanal 10,48 Gew.-% 2-Methylbutanal (entsprechend einem Molverhältnis von 100 zu 11,79) enthielt.

In die Oxidation wurden 800,0 g dieser Mischung eingesetzt.

Nach 6 Stunden Oxidation bei konstant 50°C wurden folgende Ergebnisse ermittelt:

| | |
|---|---|
| Auswaage Oxidationsprodukt (g) | 934,7 |
| GC-Analyse (%): | |
| Leichtsieder | 1,27 |
| 2-Methylbutanal | 0,15 |
| n-Pentanal | 2,14 |
| 2-Methylbuttersäure | 9,49 |
| n-Pentansäure | 86,39 |
| Hochsieder | 0,56 |
| | |
| Umsatz n-Pentanal (% d.Th.) | 97,5 |
| Selektivität zu n-Pentansäure (% d.Th.) | 99,3 |

### Beispiel 3

Dieser Versuch wurde unter den Bedingungen des Vergleichsbeispiels 1 durchgeführt mit der Abweichung, dass der Einsatzaldehyd neben 79,01 Gew.-% n-Pentanal 20,33 Gew.-% 2-Methylbutanal (entsprechend einem Molverhältnis von 100 zu 25,73) enthielt.

In die Oxidation wurden 800,0 g dieser Mischung eingesetzt.

Nach 6 Stunden Oxidation bei konstant 50°C wurden folgende Ergebnisse ermittelt:

| | |
|---|---|
| Auswaage Oxidationsprodukt (g) | 933,9 |
| GC-Analyse (%): | |
| Leichtsieder | 2,43 |
| 2-Methylbutanal | 0,25 |
| n-Pentanal | 1,85 |
| 2-Methylbuttersäure | 18,40 |
| n-Pentansäure | 76,45 |
| Hochsieder | 0,62 |
| | |
| Umsatz n-Pentanal (% d.Th.) | 97,6 |
| Selektivität zu n-Pentansäure (% d.Th.) | 99,0 |

### Beispiel 4

Dieser Versuch wurde unter den Bedingungen des Vergleichsbeispiels 1 durchgeführt mit der Abweichung, dass der Einsatzaldehyd neben 94,05 Gew.-% n-Pentanal 5,31 Gew.-% 3-Methylbutanal (entsprechend einem Molverhältnis von 100 zu 5,64) enthielt.

In die Oxidation wurden 800,0 g dieser Mischung eingesetzt.

Nach 6 Stunden Oxidation bei konstant 50°C wurden folgende Ergebnisse ermittelt:

| | |
|---|---|
| Auswaage Oxidationsprodukt (g) | 935,2 |
| GC-Analyse (%): | |
| Leichtsieder | 0,39 |
| 3-Methylbutanal | 0,20 |
| n-Pentanal | 2,67 |
| 3-Methylbuttersäure | 5,16 |
| n-Pentansäure | 91,16 |
| Hochsieder | 0,42 |
| | |
| Umsatz n-Pentanal (% d.Th.) | 97,1 |
| Selektivität zu n-Pentansäure (% d.Th.) | 99,3 |

### Beispiel 5

Dieser Versuch wurde unter den Bedingungen des Vergleichsbeispiels 1 durchgeführt mit der Abweichung, dass der Einsatzaldehyd neben 89,38 Gew.-% n-Pentanal 10,05 Gew.-% 3-Methylbutanal (entsprechend einem Molverhältnis von 100 zu 11,31) enthielt.

In die Oxidation wurden 800,0 g dieser Mischung eingesetzt.

Nach 6 Stunden Oxidation bei konstant 50°C wurden folgende Ergebnisse ermittelt:

| | |
|---|---|
| Auswaage Oxidationsprodukt (g) | 934,7 |
| GC-Analyse (%): | |
| Leichtsieder | 0,40 |
| 3-Methylbutanal | 0,38 |
| n-Pentanal | 2,39 |
| 3-Methylbuttersäure | 9,71 |
| n-Pentansäure | 86,70 |
| Hochsieder | 0,42 |
| | |
| Umsatz n-Pentanal (% d.Th.) | 97,3 |
| Selektivität zu n-Pentansäure (% d.Th.) | 99,4 |

### Beispiel 6

Dieser Versuch wurde unter den Bedingungen des Vergleichsbeispiels 1 durchgeführt mit der Abweichung, dass der Einsatzaldehyd neben 79,32 Gew.-% n-Pentanal 20,04 Gew.-% 3-Methylbutanal (entsprechend einem Molverhältnis von 100 zu 25,26) enthielt.

In die Oxidation wurden 800,0 g dieser Mischung eingesetzt.

Nach 6 Stunden Oxidation bei konstant 50°C wurden folgende Ergebnisse ermittelt:

| | |
|---|---|
| Auswaage Oxidationsprodukt (g) | 934,4 |
| GC-Analyse (%): | |
| Leichtsieder | 0,36 |
| 3-Methylbutanal | 0,67 |
| n-Pentanal | 1,80 |
| 3-Methylbuttersäure | 19,52 |
| n-Pentansäure | 77,30 |
| Hochsieder | 0,35 |
| | |
| Umsatz n-Pentanal (% d.Th.) | 97,7 |
| Selektivität zu n-Pentansäure (% d.Th.) | 99,3 |

### Herstellung von n-Heptansäure

### Vergleichsbeispiel 2

Die Flüssigphasenoxidation von n-Heptanal zu n-Heptansäure wurde ohne Katalysatorzusatz in einem Blasensäulen-Reaktor aus Glas mit einem Innendurchmesser von 38 mm und 150 cm Länge durchgeführt. Abhängig vom Reaktionsverhalten wurde der Reaktor mantelseitig durch einen mit einem Wärmeaustauscher verbundenen Wasserkreislauf gekühlt oder beheizt und die Innentemperatur auf diese Weise konstant gehalten. Die Sauerstoffzufuhr erfolgte von unten durch eine mit der Blasensäule verbundene Glasfilterplatte mit einer maximalen Porenweite von 16-40µm.

In die Oxidationen wurden jeweils 800,0 g n-Heptanal folgender, gaschromatographisch bestimmter, Zusammensetzung eingesetzt:
0,62 % Leichtsieder
97,74 % n-Heptanal
0,10 % n-Heptansäure
1,54 % Hochsieder

Nach 6 Stunden Oxidation bei konstant 50°C wurden folgende Ergebnisse ermittelt:

| | |
|---|---|
| Auswaage Oxidationsprodukt (g) | 892,0 |
| GC-Analyse (%): | |
| Leichtsieder | 0,82 |
| n-Heptanal | 5,41 |
| n-Heptansäure | 91,80 |
| Hochsieder | 1,97 |
| | |
| Umsatz n-Heptanal (% d.Th.) | 93,8 |
| Selektivität zu n-Heptansäure (% d.Th.) | 98,9 |

### Beispiel 7

Dieser Versuch wurde unter den Bedingungen des Vergleichsbeispiels 2 durchgeführt mit der Abweichung, dass der Einsatzaldehyd neben 92,86 Gew.-% n-Heptanal 4,98 Gew.-% 2-Methylhexanal (entsprechend einem Molverhältnis von 100 zu 5,36) enthielt.

In die Oxidation wurden 800,0 g dieser Mischung eingesetzt.

Nach 6 Stunden Oxidation bei konstant 50°C wurden folgende Ergebnisse ermittelt:

| | |
|---|---|
| Auswaage Oxidationsprodukt (g) | 897,3 |
| GC-Analyse (%): | |
| Leichtsieder | 1,34 |
| 2-Methylhexanal | 0,19 |
| n-Heptanal | 4,21 |
| 2-Methylhexansäure | 4,48 |
| n-Heptansäure | 88,01 |
| Hochsieder | 1,77 |
| | |
| Umsatz n-Heptanal (% d.Th.) | 94,9 |
| Selektivität zu n-Heptansäure (% d.Th.) | 98,7 |

### Beispiel 8

Dieser Versuch wurde unter den Bedingungen des Vergleichsbeispiels 2 durchgeführt mit der Abweichung, dass der Einsatzaldehyd neben 87,81 Gew.-% n-Heptanal 10,03 Gew.-% 2-Methylhexanal (entsprechend einem Molverhältnis von 100 zu 11,42) enthielt.

In die Oxidation wurden 800,0 g dieser Mischung eingesetzt.

Nach 6 Stunden Oxidation bei konstant 50°C wurden folgende Ergebnisse ermittelt:

| | |
|---|---|
| Auswaage Oxidationsprodukt (g) | 899,7 |
| GC-Analyse (%): | |
| Leichtsieder | 1,65 |
| 2-Methylhexanal | 0,41 |
| n-Heptanal | 2,80 |
| 2-Methylhexansäure | 8,86 |
| n-Heptansäure | 84,28 |
| Hochsieder | 2,00 |
| | |
| Umsatz n-Heptanal (% d.Th.) | 96,4 |
| Selektivität zu n-Heptansäure (% d.Th.) | 98,6 |

### Herstellung von n-Nonansäure

### Vergleichsbeispiel 3

Die Flüssigphasenoxidation von n-Nonanal zu n-Nonansäure wurde ohne Katalysatorzusatz in einem Blasensäulen-Reaktor aus Glas mit einem Innendurchmesser von 38 mm und 150 cm Länge durchgeführt. Abhängig vom Reaktionsverhalten wurde der Reaktor mantelseitig durch einen mit einem Wärmeaustauscher verbundenen Wasserkreislauf gekühlt oder beheizt und die Innentemperatur auf diese Weise konstant gehalten. Die Sauerstoffzufuhr erfolgte von unten durch eine mit der Blasensäule verbundene Glasfilterplatte mit einer maximalen Porenweite von 16-40µm.

In die Oxidationen wurden jeweils 800,0 g n-Nonanal folgender, gaschromatographisch bestimmter, Zusammensetzung eingesetzt:
0,33 % Leichtsieder
0,05 % 2-Methyloctanal
99,56 % n-Nonanal
0,06 % Hochsieder

Nach 6 Stunden Oxidation bei konstant 60°C wurden folgende Ergebnisse ermittelt:

| | |
|---|---|
| Auswaage Oxidationsprodukt (g) | 887,8 |
| GC-Analyse (%): | |
| Leichtsieder | 0,51 |
| n-Nonanal | 5,27 |
| 2-Methyloctansäure | 0,05 |
| n-Nonansäure | 93,32 |
| Hochsieder | 0,85 |
| | |
| Umsatz n-Nonanal (% d.Th.) | 94,2 |
| Selektivität zu n-Nonansäure (% d.Th.) | 98,8 |

### Beispiel 9

Dieser Versuch wurde unter den Bedingungen des Vergleichsbeispiels 3 durchgeführt mit der Abweichung, dass der Einsatzaldehyd neben 94,52 Gew.-% n-Nonanal 5,09 Gew.-% 2-Methyloctanal (entsprechend einem Molverhältnis von 100 zu 5,39) enthielt.

In die Oxidation wurden 800,0 g dieser Mischung eingesetzt.

Nach 6 Stunden Oxidation bei konstant 60°C wurden folgende Ergebnisse ermittelt:

| | |
|---|---|
| Auswaage Oxidationsprodukt (g) | 889,4 |
| GC-Analyse (%): | |
| Leichtsieder | 1,14 |
| 2-Methyloctanal | 0,08 |
| n-Nonanal | 3,39 |
| 2-Methyloctansäure | 4,45 |
| n-Nonansäure | 90,33 |
| Hochsieder | 0,61 |
| | |
| Umsatz n-Nonanal (% d.Th.) | 96,0 |
| Selektivität zu n-Nonansäure (% d.Th.) | 98,7 |

### Beispiel 10

Dieser Versuch wurde unter den Bedingungen des Vergleichsbeispiels 3 durchgeführt mit der Abweichung, dass der Einsatzaldehyd neben 91,30 Gew.-% n-Nonanal 8,10 Gew.-% 2-Methyloctanal (entsprechend einem Molverhältnis von 100 zu 8,87) enthielt.

In die Oxidation wurden 800,0 g dieser Mischung eingesetzt.

Nach 6 Stunden Oxidation bei konstant 60°C wurden folgende Ergebnisse ermittelt:

| | |
|---|---|
| Auswaage Oxidationsprodukt (g) | 889,8 |
| GC-Analyse (%): | |
| Leichtsieder | 1,56 |
| 2-Methyloctanal | 0,11 |
| n-Nonanal | 2,98 |
| 2-Methyloctansäure | 6,99 |
| n-Nonansäure | 87,73 |
| Hochsieder | 0,63 |
| | |
| Umsatz n-Nonanal (% d.Th.) | 96,4 |
| Selektivität zu n-Nonansäure (% d.Th.) | 98,6 |

### Beispiel 11

Dieser Versuch wurde unter den Bedingungen des Vergleichsbeispiels 3 durchgeführt mit der Abweichung, dass der Einsatzaldehyd neben 85,14 Gew.-% n-Nonanal 14,24 Gew.-% 2-Methyloctanal (entsprechend einem Molverhältnis von 100 zu 16,73) enthielt.

In die Oxidation wurden 800,0 g dieser Mischung eingesetzt.

Nach 6 Stunden Oxidation bei konstant 60°C wurden folgende Ergebnisse ermittelt:

| | |
|---|---|
| Auswaage Oxidationsprodukt (g) | 889,2 |
| GC-Analyse (%): | |
| Leichtsieder | 2,71 |
| 2-Methyloctanal | 0,15 |
| n-Nonanal | 2,51 |
| 2-Methyloctansäure | 12,63 |
| n-Nonansäure | 81,02 |
| Hochsieder | 0,98 |
| | |
| Umsatz n-Nonanal (% d.Th.) | 96,8 |
| Selektivität zu n-Nonansäure (% d.Th.) | 97,2 |

## Patentansprüche

1. Verfahren zur Herstellung geradkettiger aliphatischer Carbonsäuren mit 4 bis 11 Kohlenstoffatomen durch Oxidation der entsprechenden geradkettigen Aldehyde mit Sauerstoff oder Sauerstoff enthaltenden Gasgemischen bei 20 bis 100°C, **dadurch gekennzeichnet, dass** die Oxidation der geradkettigen Aldehyde in Gegenwart von 1 bis 30 mol-% verzweigten Aldehyden je mol geradkettigen Aldehyden erfolgt.

2. Verfahren nach Anspruch 1, **dadurch gekennzeichnet, dass** die Oxidation der geradkettigen Aldehyde in Gegenwart von 2 bis 15 mol%, insbesondere von 5 bis 10 mol-% verzweigtem Aldehyd je mol geradkettigem Aldehyd erfolgt.

3. Verfahren nach Anspruch 1 oder 2, **dadurch gekennzeichnet, dass** der verzweigte Aldehyd 3 bis 15, insbesondere 4 bis 11 Kohlenstoffatome enthält.

4. Verfahren nach einem oder mehreren der Ansprüche 1 bis 3, **dadurch gekennzeichnet, dass** sich die Seitenkette des verzweigten Aldehyds in α- oder β-Stellung zur Carbonylgruppe befindet.

5. Verfahren nach einem oder mehreren der Ansprüche 1 bis 4, **dadurch gekennzeichnet, dass** die Seitenkette ein Methylrest oder ein Ethylrest ist.

6. Verfahren nach einem oder mehreren der Ansprüche 1 bis 5, **dadurch gekennzeichnet, dass** die Oxidation bei Temperaturen im Bereich von 20 bis 80°C, vorzugsweise 40 bis 80°C durchgeführt wird.

7. Verfahren nach einem oder mehreren der Ansprüche 1 bis 6, **dadurch gekennzeichnet, dass** die Oxidation bei Drücken in einem Bereich von Atmosphärendruck bis 1,0 MPa, vorzugsweise Atmosphärendruck bis 0,8 MPa, durchgeführt wird.

8. Verfahren nach einem oder mehreren der Ansprüche 1 bis 7, **dadurch gekennzeichnet, dass** die Sauerstoff enthaltenden Gasgemische einen Anteil von bis zu 90 Vol-%, insbesondere 30 bis 80 Vol-%, inerter Bestandteile aufweisen.

## Claims

1. A process for preparing straight-chain aliphatic carboxylic acids having 4 to 11 carbon atoms by oxidation of the corresponding straight-chain aldehydes with oxygen or oxygen-containing gas mixtures at 20 to 100°C, wherein the oxidation of the straight-chain aldehydes takes place in the presence of from 1 to 30 mol-%of branched aldehydes per mol of straight-chain aldehydes.

2. The process as claimed in claim 1, wherein the oxidation of the straight-chain aldehydes takes place in the presence of from 2 to 15 mol-%, in particular from 5 to 10 mol-%, of branched aldehyde per mol of straight-chain aldehyde.

3. The process as claimed in claim 1 or 2, wherein the branched aldehyde comprises 3 to 15, in particular 4 to 11, carbon atoms.

4. The process as claimed in one or more of claims 1 to 3, wherein the side chain of the branched aldehyde is in the position α or β relative to the carbonyl group.

5. The process as claimed in one or more of claims 1 to 4, wherein the side chain is a methyl radical or an ethyl radical.

6. The process as claimed in one or more of claims 1 to 5, wherein the oxidation is carried out at temperatures in the range from 20 to 80°C, preferably 40 to 80°C.

7. The process as claimed in one or more of claims 1 to 6, wherein the oxidation is carried out under pressures in a range from atmospheric pressure to 1.0 MPa, preferably atmospheric pressure to 0.8 MPa.

8. The process as claimed in one or more of claims 1 to 7, wherein the oxygen-containing gas mixtures have a content of up to 90% by volume, in particular 30 to 80% by volume, of inert constituents.

## Revendications

1. Procédé pour la préparation d'acides carboxyliques aliphatiques linéaires comprenant 4 à 11 atomes de carbone par oxydation des aldéhydes linéaires correspondants avec de l'oxygène ou des mélanges gazeux contenant de l'oxygène à 20 jusqu'à 100°C, **caractérisé en ce que** l'oxydation des aldéhydes linéaires est réalisée en présence de 1 à 30% en mole d'aldéhydes ramifiés par mole d'aldéhydes linéaires.

2. Procédé selon la revendication 1, **caractérisé en ce que** l'oxydation des aldéhydes linéaires est réalisée en présence de 2 à 15% en mole, en particulier de 5 à 10% en mole d'aldéhyde ramifié par mole d'aldéhyde linéaire.

3. Procédé selon la revendication 1 ou 2, **caractérisé en ce que** l'aldéhyde ramifié comprend 3 à 15, en particulier 4 à 11 atomes de carbone.

4. Procédé selon l'une ou plusieurs des revendications 1 à 3, **caractérisé en ce que** la chaîne latérale de l'aldéhyde ramifié se trouve en position α ou β par rapport au groupe carbonyle.

5. Procédé selon l'une ou plusieurs des revendications 1 à 4, **caractérisé en ce que** la chaîne latérale est un radical méthyle ou éthyle.

6. Procédé selon l'une ou plusieurs des revendications 1 à 5, **caractérisé en ce que** l'oxydation est réalisée à des températures dans la plage de 20 à 80°C, de préférence de 40 à 80°C.

7. Procédé selon l'une ou plusieurs des revendications 1 à 6, **caractérisé en ce que** l'oxydation est réalisée à des pressions dans la plage allant de la pression atmosphérique à 1,0 MPa, de préférence 0,8 MPa.

8. Procédé selon l'une ou plusieurs des revendications 1 à 7, **caractérisé en ce que** les mélanges gazeux contenant de l'oxygène présentent une proportion allant jusqu'à 90% en volume, en particulier de 30 à 80% en volume de constituants inertes.
